(19) **Europäisches Patentamt**
**European Patent Office**
**Office européen des brevets**

(11) **EP 2 474 620 B1**

(12) **EUROPEAN PATENT SPECIFICATION**

(45) Date of publication and mention
of the grant of the patent:
**25.04.2018 Bulletin 2018/17**

(51) Int Cl.:
*C12Q 1/60* *(2006.01)*     *G01N 33/92* *(2006.01)*

(21) Application number: **11196013.4**

(22) Date of filing: **29.12.2011**

(54) **Method for removing high density lipoprotein in blood**

Verfahren zur Entfernung von hochdichtem Lipoprotein aus dem Blut

Procédé pour prélever de lipoprotéine haute densité dans le sang

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.01.2011 JP 2011002922
20.12.2011 JP 2011278032**

(43) Date of publication of application:
**11.07.2012 Bulletin 2012/28**

(73) Proprietor: **Fujifilm Corporation**
**Minato-ku**
**Tokyo 106-8620 (JP)**

(72) Inventors:
• **Chiku, Hiroyuki**
**Kanagawa, 258-8577 (JP)**
• **Jumawid, Mariejoy**
**Kanagawa, 258-8577 (JP)**

(74) Representative: **Hoffmann Eitle**
**Patent- und Rechtsanwälte PartmbB**
**Arabellastraße 30**
**81925 München (DE)**

(56) References cited:
**EP-A1- 0 627 627     EP-A2- 0 259 076
EP-A2- 0 602 455     JP-A- H08 113 595
US-A- 5 141 872     US-A- 5 409 840**

**Description**

**BACKGROUND OF THE INVENTION**

1. Field of the Invention

The present invention relates to a method for removing high density lipoprotein in blood, and a method for measuring low density lipoprotein in a blood sample to which the above-described method has been applied.

2. Description of the Related Art

Lipoproteins are complex particles present in circulatory systems, and are composed of proteins and lipids. One of the functions of lipoproteins is to transport water-insoluble substances such as cholesterol and cholesterol esterase so as to eventually utilize these substances in the cells. While the cells require cholesterol for the growth, when the cells accumulate excessive cholesterol, there is a possibility that certain diseases including atherosclerosis may occur.
The lipoproteins present in the blood are classified, based on their densities. Examples of the classes of the lipoproteins include very low density lipoproteins (VLDL), low density lipoproteins (LDL, a diameter of about 20 to 25 nm), and high density lipoproteins (HDL, a diameter of about 10 to 15 nm). These lipoproteins contain a different amount of cholesterol. The total serum cholesterol level can be represented by the sum of the amount of cholesterol in each of the lipoproteins.
It is known that the total serum cholesterol level is associated with the incidence of atherosclerosis. According to a recent study, it has been suggested that the lipoproteins in a certain class, as compared with the lipoproteins in the other classes, are closely associated with the progression of heart diseases including atherosclerosis. In another recent study, it has been suggested that LDL is involved as a lipoprotein class which is associated with the accumulation of cholesterol in the cells, whereas it has been revealed that HDL is involved in the removal of excess cholesterol from the cell. Therefore, methods for measuring lipoproteins, in particular LDL, have been developed. Usually, HDL and LDL are measured with a large automated analyzer provided with a liquid dispensing device. In order to measure LDL by the measuring device of the dry analytical element, lipoproteins other than LDL (particularly, HDL) are required to be removed from a blood sample.
JP1995-55812A (JP-H07-55812A) discloses that high density lipoprotein is separated from a blood sample by using a test tool having a porous silica gel having a specific particle size and surface pores. In addition, JP1994-213901A (JP-H06-213901A) discloses that the blood sample is brought into contact with the porous silica or silicate granulated as adsorbent grains to form a siliceous material gel/HDL complex, the complex is separated from the blood sample by a solid/liquid separation technique, and a blood sample containing practically no HDL is obtained. According to the prior art as described above, HDL can be adhered to pore silica by mixing the pore silica with the blood sample in an appropriate proportion.
EP 0 627 627 A1 describes a dry phase device for separating high density lipoprotein from a blood (serum or plasma) sample. The device comprises a fluid permeable material having dispersed therein finely divided; porous silica or silicate particles as selective absorbent for HDL. By combining the device with a second layer designed to remove VLDL/chylomicrons from the blood, and a third layer containing means for quantitative cholesterol detection, there is provided a test device for the direct determination of LDL cholesterol.
EP 0 602 455 A2 describes a method for separating high density lipoprotein from blood serum or plasma. The method involves contacting the blood sample with an absorbent material comprising porous silica or silicate to adsorb the high density lipoprotein in preference to other lipoproteins in the blood sample. When combined with means for removing very low density lipoproteins and chylomicrons from the blood sample, the remaining low density lipoproteins can be measured directly.
US 5 409 840 describes a process for isolating and purifying a cholesterol rich fraction from liquid mammalian blood serum or plasma containing cholesterol. The process includes contacting the cholesterol containing serum or plasma with a precipitated silica adsorbent at a pH of from 4.0 to 9.0 to adsorb a cholesterol rich fraction of the serum or plasma.
EP 0 259 076 A2 describes a method, device and kit for a quantitative determination of lipoprotein components, especially cholesterol and triglycerides, in body fluids, especially human serum, plasma or whole blood, utilizing the selective adsorption of lipoproteins on particulate silica. The method comprises contacting the sample with a solid phase comprising a quantity of particulate silica which is capable of selectively adsorbing the entire amount of lipoproteins in said sample; separating said solid phase with said lipoproteins adsorbed thereon from the non-adsorbed components of the sample; and washing said solid phase with water or an aqueous solution.
US 5 141 872 describes a method for the direct quantitative determination of LDL-cholesterol in a sample of blood plasma which involves selective adsorption of lipoproteins on silica, removal of high density lipoproteins by incubation in a suitable detergent solution, extraction of the remaining LDL-cholesterol by another detergent and determination of the LDL-cholesterol by spectrophotometric analysis.

JP H08 113595 A describes a method for purifying lipoprotein in a short time. In the method, dry or wet silica particles are admixed with a solution containing a lipoprotein such as serum to effect adsorption of the lipoprotein to the silica particles. Subsequently, the lipoprotein-adsorbed particles are collected by e.g., centrifugal separation and brought into contact with an aqueous solution of a chaotropic salt such as an aqueous sodium trichloroacetate solution to desorb the lipoprotein.

## SUMMARY OF THE INVENTION

However, the large automated analyzer as mentioned above has a disadvantage in that it is larger and more expensive, as compared with the measuring device of a dry analytical element. Moreover, there is a problem in that in a case where pore silica was added directly to the blood sample, since the adsorption efficiency of HDL to the pore silica is low, a lot of time is required. The present invention has been made to overcome the problems of the prior art described above. That is to say, the object of the present invention is to provide a method for selectively and rapidly removing high density lipoprotein (HDL) from a blood sample. Another object of the present invention is to provide a method for measuring low density lipoprotein in the blood sample to which the above-described method has been applied, by a dry analytical method.

As a result of intensive studies directed towards achieving the aforementioned object, the present inventors have found that by contacting a blood sample with a pore silica under the conditions of pH 3.0 to 6.5, high density lipoprotein (HDL) can be selectively (efficiently) and rapidly adhered to the pore silica. The present invention has been completed based on these findings.

That is to say, according to the present invention, there is provided a method for removing high density lipoprotein from a blood sample, which includes contacting the blood sample with a pore silica under the conditions of pH 5.0 to 6.0 to form a complex composed of the pore silica and the high density lipoprotein, and separating the complex from the blood sample.

The blood sample is brought into contact with the pore silica for 5 minutes or less.

Preferably, the pore silica is in the form of powder, dispersion, or solid.

Preferably, the pore silica has an average particle diameter of 10 to 100 $\mu$m, a micropore diameter of 10 to 200 nm, and a specific surface area of 10 to 200 m$^2$/g.

According to the present invention, there is further provided a method for measuring low density lipoprotein in a blood sample, which comprises removing high density lipoprotein from a blood sample by the method of the present invention described above, and measuring the low density lipoprotein in the blood sample from which the high density lipoprotein has been removed by using (a) cholesterol esterase, and (b) cholesterol oxidase or cholesterol dehydrogenase.

Preferably, the low density lipoprotein cholesterol is measured by allowing peroxidase and a chromogen to act on hydrogen peroxide generated from the low density lipoprotein cholesterol by cholesterol esterase and cholesterol oxidase, so as to carry out a color development reaction.

Preferably, the low density lipoprotein in the blood sample is measured by using a dry analytical element for measuring low density lipoprotein cholesterol, which comprises (a) cholesterol esterase, (b) cholesterol oxidase, (c) peroxidase, and (d) a chromogen.

According to the present invention, high density lipoprotein (HDL) can be selectively, rapidly, and easily removed from the blood sample. Low density lipoprotein (LDL) in the blood can be rapidly measured with the dry analytical element by using the present sample.

## DESCRIPTION OF THE PREFERRED EMBODIMENTS

Hereinafter, the embodiments of the present invention will be further described in detail.

The present invention relates to a method for removing high density lipoprotein from a blood sample, which comprises contacting the blood sample with a pore silica under the conditions of pH 5.0 to 6.0 to form a complex composed of the pore silica and the high density lipoprotein, and separating the complex from the blood sample, wherein the blood sample is brought into contact with the pore silica for 5 minutes or less. The present invention further relates to a method for measuring low density lipoprotein in a blood sample, which comprises removing high density lipoprotein from the blood sample by the aforementioned method, and measuring the low density lipoprotein in the blood sample from which the high density lipoprotein has been removed by using (a) cholesterol esterase, and (b) cholesterol oxidase or cholesterol dehydrogenase.

As a blood sample, blood (whole blood), serum, or plasma, and the like can be used. As the blood sample, blood (whole blood), serum, or plasma, and the like may be directly used, or otherwise the blood, serum, or plasma, and the like, which has been pre-treated as appropriate, may also be used.

In the present invention, the blood sample is brought into contact with the pore silica under the conditions of pH 5.0 to 6.0. The conditions of pH 5.0 to 6.0 lead to the attainment of the object of the present invention that high density lipoprotein (HDL) can be selectively and rapidly removed from the blood sample.

While the form of the pore silica used in the present invention is not particularly limited, it is generally in the form of powder, dispersion, or solid. While pure silica consists of silicon and oxygen, the silica to which metals, metal oxides, or metal salts have been added is sometimes called silica. In addition, high density lipoprotein (HDL) can interact with the silica having a greater average pore diameter than the diameter of HDL particle. It is preferable that the pore size of the pore silica be small enough to reduce the interaction of the silica with large lipoprotein particles such as LDL or VLDL. Microporous pore silica can be obtained, for example, by heating hydrated silica gel at 1,000°C for about 10 hours.

While the average particle diameter of the pore silica used in the present invention is not particularly limited, it is preferably 10 to 100 $\mu$m. While the micropore diameter of the pore silica is not particularly limited, it is preferably 10 to 200 nm, and more preferably 20 to 150 $\mu$m. While the specific surface area of the pore silica is not particularly limited, it is preferably 10 to 200 $m^2$/g, and more preferably 30 to 170 $m^2$/g. In addition, the average particle diameter can be measured by a laser diffraction and scattering method (laser diffraction/scattering particle size distribution analyzer, Partica LA-950V2, manufactured by HORIBA, Ltd.), and the like. Additionally, the micropore diameter can be measured by a mercury intrusion method (fully automatic pore size distribution analyzer, Pore Master 60-GT, manufactured by Quantachrome), and the like. Further, the specific surface area can be measured by a Brunauer-Emmett-Teller (BET) method, and the like.

While the amounts of the pore silica used can be appropriately determined depending on the kinds of the pore silica, and the like, the final concentration of the pore silica in the mixed state with the blood sample is 10 to 1000 mg/ml, preferably 20 to 500 mg/ml.

The blood sample may be suspended only for a given period, wherein the pore silica is dispersed in an appropriate buffer solution to prepare a silica dispersion, and the silica dispersion is mixed with the blood sample. While time for suspending is not particularly limited as far as the object of the present invention can be attained, the lower limit is preferably 1 second or more, more preferably 10 seconds or more, and further preferably 30 seconds or more, and the upper limit is 5 minutes or less, and preferably 3 minutes or less. The contact of the blood sample with the pore silica can be performed with a conventional mixing operation such as inversion-mixing, vortexing, and pipetting. As a buffer solution which is used to adjust the silica dispersion, this is not particularly limited as far as the buffer solution has a buffering action at pH 5.0 to 6.0, and for example, citric acid, phosphoric acid, and the like can be used.

In the present invention, the low density lipoprotein in a blood sample obtained from the aforementioned method can be measured by using (a) cholesterol esterase, and (b) cholesterol oxidase or cholesterol dehydrogenase. As cholesterol esterase, lipase can also be used. While types of microorganisms, from which such enzymes are derived, are not particularly limited, for example, with regard to cholesterol esterase, *Schizophyllum commune*-derived or *Pseudomonas* sp.-derived esterase, or other types of microorganisms-derived esterases, and the like can be used. With regard to cholesterol oxidase, *Pseudomonas* sp.-derived, *Streptomyces* sp.-derived, or other types of microorganism-derived oxidases, and the like can be used. The enzyme used in the present invention may be either an enzyme derived from such microorganisms, or a recombinant enzyme produced by a well-known method.

An example of the cholesterol esterase derived from *Schizophyllum commune* is COE-302 manufactured by Toyobo Co., Ltd., and examples of the cholesterol esterase derived from *Pseudomonas* sp. include COE-311, LPL-312, and LPL-314 manufactured by Toyobo Co., Ltd., and CEN manufactured by Asahi Kasei Corporation, and the like. Examples of the cholesterol oxidase derived from *Pseudomonas* sp. include CHO-PEL and CHO-PEWL manufactured by Kikkoman Corporation, and the like.

In the method of the present invention, in addition to these reagents, as reagents for detecting cholesterol, an enzyme reagent, a chromogen, and a pH buffer, which are well known, can be used.

More specifically, an example of the enzyme can be peroxidase, and examples of the chromogen can include 4-aminoantipyrine (4-AA), a phenolic or anilinic Trinder's reagent that develops color as a result of hydrogen-donating coupling therewith, and a leuco dye, and the like. As the Trinder's reagent, an aniline reagent can be preferably used. Examples of aniline Trinder's reagent include N-ethyl-N-sulfopropyl-3-methoxyaniline (ADPS), N-ethyl-N-sulfopropylaniline (ALPS), N-ethyl-N-sulfopropyl-3-methylaniline (TOPS), N-ethyl-N-(2-hydroxy-3-sulfopropyl)-3-methoxyaniline (ADOS), N-ethyl-N-(2-hydroxy-3-sulfopropyl)-3,5-dimethoxyaniline (DAOS), N-(2-hydroxy-3-sulfopropyl)-3,5-dimethoxyaniline (HDAOS), N-ethyl-N-(2-hydroxy-3-sulfopropyl)-3,5-dimethylaniline (MAOS), and N-ethyl-N-(2-hydroxy-3-sulfopropyl)-3-methoxyaniline (TOOS), and the like, manufactured by Dojindo Laboratories.

Examples of a pH buffer include a carbonate, a sulfate, a phosphate, and a Good's pH buffer described in Biochemistry, 5, pp. 467-477, 1966. These pH buffers can be selected with reference to the descriptions of publications, such as Basic Experimental Methods for Proteins and Enzymes, Takeichi Horio et al., Nankodo Co., Ltd., 1981, and Biochemistry, 5, pp. 467-477, 1966.

While the pH of the above-mentioned buffer can be determined depending on the optimal pH of enzyme used, it can be adjusted to preferably the range of from pH 5.0 to 8.0, and more preferably the range of from pH 6.0 to 7.0.

While the measurement method of the present invention in a case where a measurement system is a solution will be described below, the specific embodiment mentioned below is not intended to limit the scope of the present invention. A reagent solution preferably has a composition containing the following (1) to (5):

(1) Cholesterol esterase
(2) Cholesterol oxidase
(3) Peroxidase
(4) Chromogens (4-AA and a Trinder's reagent)
(5) pH buffer.

1 to 1,000 μL of, and preferably 100 to 500 μL of a reagent solution wherein the concentrations of the above-described reagents had been adjusted to optimal concentrations is pre-incubated for 1 to 10 minutes, at a constant temperature in the range of from approximately 20°C to approximately 45°C, and preferably in the range of from approximately 30°C to approximately 40°C. Thereafter, 0.5 to 50 μL of, and preferably 1 to 20 μL of a solution sample is added to the above-described reagent solution, and while incubating at a constant temperature, a change over time in wavelength due to the color development of the chromogens is measured. Using a previously prepared calibration curve, the amount of a test substance in a specimen can be determined in accordance with the principle of colorimetric methods.

While the necessary amounts of enzymes can be appropriately determined, for example, any enzyme of cholesterol esterase, cholesterol oxidase and peroxidase can be used, preferably within the range of from 0.2 to 500 U/mL, more preferably within the range of from 0.2 to 100 U/mL, and further preferably within the range of from 1 to 50 U/mL.

When the measurement system is a solution, one or two or more types of surfactants may be used in combination with them, as necessary. While the concentration of surfactant is not particularly limited, for example, the surfactant can be used at a concentration of preferably 0.01% to 20%, and more preferably 0.1% to 15%.

The measurement method of the present invention using a dry analytical element, in which the measurement system is a dry reagent, will be described. The dry analytical element can be configured to have at least one adhesion layer and a porous spreading layer on a water-impermeable support.

The porous layer may be made of either a fibrous or non-fibrous material, and since such a porous layer functions as a layer for spreading a liquid sample, it is preferably a layer having a liquid measuring action. The liquid measuring action means an action to extend a liquid sample spotted to the surface of the layer in a nearly constant proportion per unit area in the surface direction of the layer substantially without uneven distribution of ingredients contained therein. In order to control the spreading area, the spreading rate, and the like, the hydrophilic polymer or surfactant described in JP1985-222770A (JP-S60-222770A), JP1988-219397A (JP-S63-219397A), and JP1987-182652A (JP-S62-182652A) can be mixed into the spreading layer, and a polycyclic polyglycidol compound as a surfactant can be also mixed into the spreading layer.

A fibrous porous layer is preferably made of polyester fibers, including those described in JP1980-164356A (JP-S55-164356A), JP1982-66359A (JP-S57-66359A) and JP1985-222769A (JP-S60-222769A), and the like, as typical examples. A non-fibrous porous layer is preferably made of an organic polymer such as polysulfonic acid.

An adhesion layer is a layer having a function of adhering the above-mentioned water-impermeable support to the above-mentioned porous layer, and examples of such adhesion layer that can be used herein include hydrophilic polymers such as gelatin and the derivatives thereof (e.g. phthalate gelatin), cellulose derivatives (e.g. hydroxypropyl cellulose), agarose, acrylamide polymers, methacrylamide polymers, and copolymers of acrylamide or methacrylamide with various types of vinyl monomers.

While an aqueous solution containing a hydrophilic polymer is uniformly coated by a well-known method, known methods for coating such an aqueous solution can be used. A coating method can be used, which is appropriately selected from dip coating, extruding coating, doctor coating, hopper coating, curtain coating, and the like, for example.

While the porous layer can also be coated onto the adhesion layer, it is preferable to laminate a cloth or a porous membrane that has previously been supplied as a fabric on the adhesion layer. According to a lamination method, as described in JP1980-164356A (JP-S55-164356A), the cloth or the membrane can be adhered to the adhesion layer by uniformly moistening the surface of an adhesion layer containing a hydrophilic polymer with water, and then by laminating the cloth or the porous membrane thereon, and thereafter, by pressing slightly and almost uniformly thereon so as to adhere the cloth or porous membrane to the adhesion layer. The thickness of such an adhesion layer is preferably 0.5 to 50 μm, and more preferably 1 to 20 μm.

Preferred materials for a light-permeable support include cellulose ethers such as polyethylene terephthalate, polystyrene, and cellulose triacetate. In order to strongly adhere a water-absorbing layer as a hydrophilic layer, a detection layer, a substantially non-porous reagent layer, and the like to a support, generally, an undercoating layer can be established on the support, or a hydrophilic treatment can be performed thereon. While the thickness of the support is not particularly limited, it is preferably 10 to 1,000 μm, and more preferably 300 to 800 μm. In the case of a light-permeable support, the final detection may be carried out either on the support side or the porous layer side, whereas, in the case of a light-impermeable support, the final detection is carried out on the porous layer side.

As necessary, a stabilizer, a pH buffer, a cross-linker (a hardener or a curing agent), a surfactant, a polymer, and the like can be contained. These agents can be contained in the adhesion layer or the porous layer.

The preferred layer-constitution of the dry analytical element for LDL-C detection will be described below.

The dry analytical element for LDL-C detection comprises an adhesion layer and a porous layer, which are coated with an aqueous solution of gelatin. The adhesion layer contains gelatin, peroxidase, and the reagent of color material. The top of the adhesion layer is laminated with a cloth or a membrane. On the cloth or the membrane, cholesterol esterase, and cholesterol oxidase or cholesterol dehydrogenase, a surfactant, and a pH buffer are coated.

The reagent compositions used for the measurement of cholesterol and the reagent compositions that bring about an optical change, which are used for a dry analytical element, will be described.

While the reagent compositions may be contained in a first porous layer, such reagent compositions may also be contained in both an adhesion layer and a porous layer. Otherwise, all or most of the reagent compositions may be contained in any of such layers, or the reagent compositions may also previously be added to any layer other than the adhesion layer and the porous layer.

In a dry analytical element used for LDL-C detection, any enzymes of cholesterol esterase, and cholesterol oxidase or cholesterol dehydrogenase, may be used in an amount of preferably approximately 0.1 to 30 kU per 1 $m^2$, and more preferably approximately 0.5 to 15 kU per 1 $m^2$.

Any of the surfactants may be used in an amount of approximately 0.2 to 50 g per 1 $m^2$, and more preferably approximately 1 to 50 g per 1 $m^2$.

While the origin of peroxidase is not particularly limited, horseradish-derived peroxidase is preferable. Peroxidase may be used in an amount of preferably approximately 1 to 200 kU/ $m^2$, and more preferably approximately 10 to 100 kU/ $m^2$.

With regard to the above-mentioned chromogen, the combination of the above-mentioned reagent that couples with 4-aminoantipyrine (4-AA) to develop color is preferable, and particularly preferably, N-ethyl-N-(2-hydroxy-3-sulfopropyl)-3-methoxyaniline (TOOS) can be used. While the amount of chromogen used is not particularly limited, for example, 4-AA and a hydrogen-donating coupling agent may be used together in an amount of preferably approximately 0.1 to 10 g/ $m^2$, and more preferably approximately 0.1 to 5 g/ $m^2$.

In the other reagent compositions in the dry analytical element used for the detection of LDL-C, one or two or more types of additives such as a stabilizer, a pH buffer, a cross-linker (a hardener or a curing agent), a surfactant and a polymer, as necessary, can be contained. These additives may be contained in the adhesion layer and/or porous layer of the dry analytical element.

The pH of a buffer can be determined depending on the optimal pH of enzyme used, and can be adjusted to preferably the range of from pH 5.0 to 8.0, and more preferably the range of from pH 6.0 to 7.0.

The dry analytical element can be cut into, for example, small pieces such as a square of having a side of approximately 5 mm to approximately 30 mm or a circular form having almost the same size, and it can be then placed in a slide frame described in JP1982-283331B (JP-S57-28333B), JP1981-142454U (JP-S56-142454U), JP1982-63452A (JP-S57-63452A), JP1983-32350U (JP-S58-32350U), JP1983-501144A (JP-S58-501144A), and the like, and then it can be used as a chemical analysis slide. This embodiment is preferable from the viewpoint of production, packaging, transport, storage, measuring operation, and the like. Depending on the purpose of usage, the dry analytical element can also be placed in a long tape form in a cassette or magazine for use, alternatively, the small piece thereof can also be placed in a container with an opening for use, or can also be attached to or placed in an opening card for use, and moreover, the small cut piece can also be used directly.

When the dry analytical element is used, a blood sample can be spotted in a range of, for example, approximately from 2 $\mu$L to approximately 30 $\mu$L, and preferably from 4 $\mu$L to 15 $\mu$L, onto the porous liquid sample spreading layer, and thereafter, the spotted dry analytical element can be incubated at a constant temperature ranging from approximately 20°C to approximately 45°C, preferably from approximately 30°C to approximately 40°C, for 1 to 10 minutes. Color development or discoloration within the dry analytical element can be reflex-measured from the light-permeable support side, and using the previously prepared calibration curve, the amount of a test substance in a specimen can be determined in accordance with the principle of colorimetric methods.

The measuring operation can be carried out extremely easily using the chemical analyzers described in JP1985-125543A (JP-S60-125543A), JP1985-220862A (JP-S60-220862A), JP1986-294367A (JP-S61-294367A), and JP1983-161867A (JP-S58-161867A), and the like, thereby performing quantitative analysis with high precision. Depending on purposes and necessary precision, the degree of color development may be assessed by visual observation to perform semi-quantitative measurement.

The dry analytical element can be stored and preserved in a dry state until the analysis is performed, and thus the reagents do not have to be prepared before use, and moreover, since reagents in a dry state generally have high stability, the method of the present invention is more convenient and rapid than a so-called solution method in which reagent solutions must be prepared before use. Moreover, the method of the present invention is also excellent as an examination method capable of rapid examination with high precision using a trace amount of liquid sample.

While the present invention will be more specifically described in the following examples, these examples are not intended to limit the scope of the present invention.

EXAMPLES

Example I: Removal of high density lipoprotein (HDL) in a blood sample by pore silica

In 115 μL of 0.1M buffer (the kinds of buffer are as described in Tables 2-1, 2-2, 2-3, 3, or 4) certain pore silicas described in Table 1 (the amounts of the pore silica are described in Tables 2-1, 2-2, 2-3, 3, or 4) were dispersed to produce silica dispersions. The pH of the samples produced here are as described in Tables 2-1, 2-2, 2-3, 3, or 4.

115 μL of serum sample was added to this silica dispersion, and thereafter suspended only for the given time (as in Tables 2-1, 2-2, 2-3, 3, or 4), and the silica was precipitated at 3,000 rpm for 30 seconds, and the concentrations of HDL and LDL contained in the supernatant were measured with an Hitachi Automatic Analyzer H7180 ([measuring reagent] HDL: determiner L HDL-C (manufactured by Kyowa Medics), LDL: determiner L LDL-C (manufactured by Kyowa Medics)). The amounts of HDL and LDL contained in the serum sample after the treatment with pore silica, relative to the amounts of HDL and LDL contained in untreated serum sample were each set as remaining HDL% and remaining LDL%. At this time, in the case where the remaining LDL%/the remaining HDL% (in the tables, it is described as a LDL/HDL calculation ratio) is 5.0 or more, it is considered to be superior to the case of the LDL measurement from this point onwards.

The results of the above-described measurements are shown in Tables 2-1, 2-2, 2-3, 3, or 4.

In addition, the average particle diameter of each compound was measured by a laser diffraction and scattering method using a laser diffraction/scattering particle size distribution analyzer (Partica LA-950V2, manufactured by HORIBA. Ltd.). First, as a pre-treatment of the samples, six samples for each compound were prepared, respectively, and ultrasonic (homogenizer) treatment in the device was run for 1 minute, for each compound. Then, under the measurement conditions of measurement mode: manual flow cell measurement, measurement range: 0.01 μm to 3000 μm, dispersion medium: 180 ml ion-exchange water, and refractive index: quartz 1.45 to 0.00i/ion-exchange water 1.33 to 0.00i, the dispersion medium was introduced to the measured cells and a blank measurement was performed, and thereafter, each sample was introduced to the quartz cell and the measurement was performed twice.

Additionally, the micropore diameter can be measured by a mercury intrusion method using fully an automatic pore size distribution analyzer (Pore Master 60-GT, manufactured by Quantachrome). First, six samples of 0.2 g to 0.4 g for each compound were prepared, respectively. Then, under the measurement conditions of measurement range: high pressure-area, sample cell: small cell 10φ × 30 mm, measurement range: micropore diameter 0.0036 μm to 10 μm, calculation range: micropore diameter 0.0036 μm to 10 μm, mercury contact angle: 140°, and mercury surface tension: 480 dyn/cm, measurement for each sample was performed. At this time, together with the measurement of the micropore diameter, the measurement of the specific surface area was performed by the BET method under the same conditions.

Table 1

| Compound name | Manufacturer | Average particle size (μm) | Micropore diameter (nm) | Specific ($m^2$/g) surface area |
|---|---|---|---|---|
| EP-DF-10-500A | AGC Si-Tech | 10.5 | 35.2 | 155 |
| D-50-500AW | | 48.0 | 47.1 | 94 |
| EP-DM-20-1000AW | | 21.6 | 117.0 | 38 |
| D-50-700AW | | 48.4 | 72.5 | 43 |
| Controlled pore glass350400 | Sigma | 72.0 | 45.8 | 103 |
| Controlled pore glass CPG500C | MILLIPORE | 67.6 | 53.1 | 92 |
| MB300 100-200 | Fuji Silysia | 96.9 | 30.1 | 168 |

Table 2-1

| | Compound name | Amount of Pore silica (mg) | Buffer | pH of sample | Pore silica/ Sample suspension time | Remaining LDL% | Remaining HDL% | LDL/HDL calculation ratio |
|---|---|---|---|---|---|---|---|---|
| Comparative Example 1 | EP-DF-10-500A | 8.0 | glycine | 2.5 | 1.0 | 47.3% | 9.8% | 4.8 |
| Reference Example 1 | | | citric acid | 3.0 | | 62.3% | 10.3% | 6.0 |
| Reference Example 2 | | | citric acid | 4.0 | | 69.8% | 11.3% | 6.2 |
| Example 3 | | | citric acid | 5.2 | | 74.1% | 11.9% | 6.2 |
| Example 4 | | | citric acid | 6.0 | | 80.1% | 13.1% | 6.1 |
| Reference Example 5 | | | phosphoric acid | 6.5 | | 88.6% | 15.2% | 5.8 |
| Comparative Example 2 | | | phosphoric acid | 7.5 | | 98.7% | 25.4% | 3.9 |
| Comparative Example 3 | | | without | 7.4 | | 95.3% | 24.4% | 3.9 |

Table 2-2

| | Compound name | Amount of Pore silica (mg) | Buffer | pH of sample | Pore silica/ Sample suspension time | Remaining LDL% | Remaining HDL% | LDL/HDL calculation ratio |
|---|---|---|---|---|---|---|---|---|
| Comparative Example 4 | D-50-500AW | 22.2 | glycine | 2.0 | 1.0 | 42.1% | 8.7% | 4.8 |
| Reference Example 6 | | | citric acid | 3.0 | | 55.0% | 9.0% | 6.1 |
| Reference Example 7 | | | citric acid | 4.0 | | 60.1% | 9.7% | 6.2 |
| Example 8 | | | citric acid | 5.2 | | 70.6% | 10.0% | 7.1 |
| Example 9 | | | citric acid | 6.0 | | 80.2% | 13.6% | 5.9 |
| Reference Example 10 | | | phosphoric acid | 6.5 | | 100.0% | 17.4% | 5.7 |
| Comparative Example 5 | | | phosphoric acid | 7.5 | | 100.0% | 25.2% | 4.0 |
| Comparative Example 6 | | | without | 7.4 | | 92.6% | 43.1% | 2.1 |

Table 2-3

| | Compound name | Amount of Pore silica (mg) | Buffer | pH of sample | Pore silica/ Sample suspension time | Remaining LDL% | Remaining HDL% | LDL/HDL calculation ratio |
|---|---|---|---|---|---|---|---|---|
| Comparative Example 7 | EP-DM-20-1000AW | 50.0 | glycine | 2.5 | 1.0 | 28.1% | 6.6% | 4.3 |
| Reference Example 11 | | | citric acid | 3.0 | | 39.9% | 7.2% | 5.5 |
| Reference Example 12 | | | citric acid | 4.0 | | 40.5% | 7.9% | 5.1 |
| Example 13 | | | citric acid | 5.2 | | 42.1% | 8.5% | 5.0 |
| Example 14 | | | citric acid | 6.0 | | 50.8% | 10.1% | 5.0 |
| Reference Example 15 | | | phosphoric acid | 6.5 | | 63.2% | 12.7% | 5.0 |
| Comparative Example 8 | | | Phosphoric acid | 7.5 | | 80.4% | 18.9% | 4.3 |
| Comparative Example 9 | | | without | 7.4 | | 79.9% | 18.5% | 4.3 |

EP 2 474 620 B1

From the results of the above-described Reference Examples 1, 2, 5-7, 10-12 and 15, Examples 3, 4, 8, 9, 13 and 14 and Comparative Examples 1 to 9, it was found that performing the contact of the pore silica with HDL at pH 3.0 to 6.5, preferably 3.0 to 6.0, and particularly preferably 4.0 to 6.0 is effective for the selective removal of high density lipoprotein (HDL).

Table 3

| | Compound name | Amount of Pore silica (mg) | Buffer | pH of sample | Pore silica/ Sample suspension time | Remaining LDL% | Remaining HDL% | LDL/HDL calculation ratio |
|---|---|---|---|---|---|---|---|---|
| Example 1 | EP-DF-10-500A | 8.0 | citric acid | 3.0 | 1.0 | 62.3% | 10.3% | 6.0 |
| Example 4 | | | citric acid | 6.0 | 1.0 | 80.1% | 13.1% | 6.1 |
| Comparative Example 10 | | | without | 7.4 | 8.0 | 70.2% | 14.4% | 4.9 |

From the results of the above-described Examples 1 and 4, and Comparative Example 10, it was found that the suspension time can be shortened by adjusting pH wherein the pore silica is brought into contact with the samples (Table 3).

Table 4

| | Compound name | Amount of Pore silica (mg) | Buffer | pH of sample | Pore silica/ Sample suspension time | Remaining LDL% | Remaining HDL% | LDL/HDL calculation ratio |
|---|---|---|---|---|---|---|---|---|
| Example 3 | EP-DF-10-500A | 8.0 | citric acid | 5.2 | 1.0 | 74.1% | 11.9% | 6.2 |
| Example 8 | D-50-500AW | 22.2 | citric acid | 5.2 | 1.0 | 70.6% | 10.0% | 7.1 |
| Example 13 | EP-DM-20-1000A W | 50.0 | citric acid | 5.2 | 1.0 | 42.1% | 8.5% | 5.0 |
| Example 16 | Controlled pore glass350400 | 22.2 | citric acid | 5.2 | 1.0 | 61.5% | 10.0% | 6.2 |
| Example 17 | Controlled pore glass CPG500C | 22.2 | citric acid | 5.2 | 1.0 | 50.0% | 5.9% | 8.5 |
| Example 18 | MB300 100-200 | 65.0 | citric acid | 5.2 | 1.0 | 92.5% | 18.3% | 5.1 |
| Example 19 | D-50-700AW | 27.0 | citric acid | 5.2 | 1.0 | 90.0% | 15.0% | 6.0 |

From the results of Examples 3, 8, 13, 16, 17, 18, and 19, it was found that high density lipoprotein (HDL) can be selectively removed by using the pore silica which has an average particle size of 10 to 100 $\mu$m, and a micropore diameter of 10 to 200 nm.

Example II: Measurement of LDL-C using Multilayer Analytical Element for Measuring LDL-C

The result of measurement of LDL-C in the blood using the multilayer analytical element is shown below.
A gelatin aqueous solution was coated on a gelatin-undercoated polyethylene terephthalate film of 180 $\mu$m in thickness which was colorless, transparent and smooth, so as to have a thickness of 14 $\mu$m after drying, followed by drying. Thereafter, the aqueous solution with the following composition was coated, followed by drying.

| | |
|---|---|
| 4-aminoantipyrine | 0.32 g/m$^2$ |
| TOOS (manufactured by Dojindo Laboratories) | 0.62 g/m$^2$ |
| Peroxidase (manufactured by Toyobo Co., Ltd.) | 12.75 kU/m$^2$ |

Water was supplied over the above film in a volume of approximately 30 g/ m$^2$ so as to moisten the film. Tricot knitted fabric prepared by knitting 36 gauge with polyester spun yarn corresponding to 50 deniers was laminated thereon via light pressurization, followed by drying. Thereafter, the aqueous solution with the following composition was coated to the above fabric, followed by drying.

| | |
|---|---|
| MOPS (pH 7.0) | 1.6 g/m$^2$ |
| Polyglyceryl tristyl phenyl ether | 5.02 g/m$^2$ |
| Pluronic L121 | 39.75 g/ m$^2$ |
| Cholesterol esterase (lipoprotein lipase, Toyobo) | 0.65 kU/ m$^2$ |
| Cholesterol oxidase (recombinant E. coli, Kikkoman) | 0.13kU/ m$^2$ |

The serum was suspended in the following silica dispersion and centrifuged for 30 seconds at 3000rpm, and the specimen was separated from the silica. The buffer of the silica dispersion is as shown in Table 5.

| | |
|---|---|
| serum | 100 $\mu$L |
| silica dispersion (222.2 mg/ml) | 100 $\mu$L |

(D-50-500AW (AGC Si-Tech) in 0.5M buffer)

The separated specimen was used, and this was spotted to the above-described multilayer analytical element, and the multi-specimen correlation was examined by the method of the present invention and the method of direct measurement for fluids with an Hitachi Automatic Analyzer H7180 (untreated serum was measured with measuring reagent: determiner LDL-C (manufactured by Kyowa Medics)). The results are shown in Table 5. The correlation formula of the measuring value X of LDL-C by the method of direct measurement for fluids and the measuring value Y of LDL-C by the method of the present invention isY= AX+B. In this case, when correlation coefficient R is 0.98 or more, it can be said that there is sufficient correlation with regard to both testing methods. Herein, the correlation coefficient R is as defined as below. When the data row consisting of two sets of data values (x, y) ={(Xi, Yi)} (i=1,2,...,n) is given, the correlation coefficient is obtained as shown below.

[formula]

$$R = \frac{\sum_{i=1}^{n}(Xi - x')(Yi - y')}{\sqrt{\sum_{i=1}^{n}(Xi - x')^2}\sqrt{\sum_{i=1}^{n}(Yi - y')^2}}$$

(with the proviso that x' and y' are an arithmetic average of all the data items x ={Xi} and y ={Yi} .)
As seen from the results of Examples 20 to 24, and Comparative Examples 11 to 13, by performing the reaction of the pore silica with serum at pH 3.0 to 6.5, preferably 3.0 to 6.0, and particularly preferably 4.0 to 6.0, the measurement for LDL-C can be performed, which has a high correlation with the method of direct measurement for liquids, even in the multilayer analytical element (Table 5).

Table 5

|  | Compound name | Buffer | pH of sample | Correlation coefficient R |
|---|---|---|---|---|
| Comparative Example 11 | D-50-500AW | glycine | 2.0 | 0.970 |
| Example 20 | | citric acid | 3.0 | 0.981 |
| Example 21 | | citric acid | 4.0 | 0.982 |
| Example 22 | | citric acid | 5.2 | 0.983 |
| Example 23 | | citric acid | 6.0 | 0.982 |
| Example 24 | | phosphoric acid | 6.5 | 0.980 |
| Comparative Example 12 | | phosphoric acid | 7.5 | 0.965 |
| Comparative Example 13 | without | citric acid | 5.2 | 0.942 |

**Claims**

1. A method for removing high density lipoprotein from a blood sample, comprising:

   contacting the blood sample with a pore silica under the conditions of pH 5.0 to 6.0 to form a complex composed of the pore silica and the high density lipoprotein; and
   separating the complex from the blood sample,
   wherein the blood sample is brought into contact with the pore silica for 5 minutes or less.

2. The method according to claim 1, wherein the pore silica is in the form of powder, dispersion, or solid.

3. The method according to claim 1 or 2, wherein the pore silica has an average particle diameter of 10 to 100 $\mu$m, a micropore diameter of 10 to 200 nm, and a specific surface area of 10 to 200 m$^2$/g.

4. A method for measuring low density lipoprotein in a blood sample, comprising:

   removing high density lipoprotein from a blood sample by the method according to any one of claims 1 to 3, and measuring the low density lipoprotein in the blood sample from which the high density lipoprotein has been removed by using (a) cholesterol esterase, and (b) cholesterol oxidase or cholesterol dehydrogenase.

5. The method according to claim 4, wherein low density lipoprotein cholesterol is measured by allowing peroxidase and a chromogen to act on hydrogen peroxide generated from the low density lipoprotein cholesterol by cholesterol esterase and cholesterol oxidase, so as to carry out a color development reaction.

6. The method according to claim 4 or 5, wherein the low density lipoprotein in the blood sample is measured by using a dry analytical element for measuring low density lipoprotein cholesterol, which comprises (a) cholesterol esterase, (b) cholesterol oxidase, (c) peroxidase, and (d) a chromogen.

**Patentansprüche**

1. Verfahren zur Entfernung von Lipoprotein hoher Dichte aus einer Blutprobe, umfassend:

   Kontaktieren der Blutprobe mit porösem Silika unter den Bedingungen eines pH-Werts von 5,0 bis 6,0 zur Bildung eines Komplexes, der aus dem porösen Silika und dem Lipoprotein hoher Dichte zusammengesetzt ist, und
   Abtrennen des Komplexes von der Blutprobe,

wobei die Blutprobe mit dem porösen Silika für 5 Minuten oder weniger in Kontakt gebracht wird.

2. Verfahren gemäß Anspruch 1, worin das poröse Silika in Form eines Pulvers, einer Dispersion oder eines Feststoffes vorliegt.

3. Verfahren gemäß Anspruch 1 oder 2, worin das poröse Silika einen durchschnittlichen Partikeldurchmesser von 10 bis 100 $\mu$m, einen Mikroporendurchmesser von 10 bis 200 nm und eine spezifische Oberfläche von 10 bis 200 m$^2$/g aufweist.

4. Verfahren zur Messung von Lipoprotein niedriger Dichte in einer Blutprobe, umfassend:

Entfernen von Lipoprotein hoher Dichte aus einer Blutprobe durch das Verfahren gemäß irgendeinem der Ansprüche 1 bis 3 und
Messen von Lipoprotein niedriger Dichte in der Blutprobe, von der das Lipoprotein hoher Dichte entfernt wurde, unter Verwendung von (a) Cholesterinesterase und (b) Cholesterinoxidase oder Cholesterindehydrogenase.

5. Verfahren gemäß Anspruch 4, worin lipoproteinisches Cholesterin niedriger Dichte gemessen wird, indem man Peroxidase und ein Chromogen auf Wasserstoffperoxid, das aus dem lipoproteinischem Cholesterin niedriger Dichte durch Cholesterinesterase und Cholesterinoxidase erzeugt wurde, so einwirken lässt, dass eine Farbentwicklungsreaktion durchgeführt wird.

6. Verfahren gemäß Anspruch 4 oder 5, worin das Lipoprotein niedriger Dichte in der Blutprobe unter Verwendung eines trockenen analytischen Elements zur Messung von lipoproteinischem Cholesterin niedriger Dichte gemessen wird, das (a) Cholesterinesterase, (b) Cholesterinoxidase, (c) Peroxidase und (d) ein Chromogen umfasst.

## Revendications

1. Procédé pour extraire la lipoprotéine de haute densité d'un échantillon de sang, comprenant:

la mise en contact de l'échantillon de sang avec une silice poreuse dans les conditions d'un pH de 5,0 à 6,0 pour former un complexe composé de la silice poreuse et de la lipoprotéine de haute densité ; et
la séparation du complexe de l'échantillon de sang,
dans lequel l'échantillon de sang est mis en contact avec la silice poreuse pendant 5 minutes ou moins.

2. Procédé selon la revendication 1, dans lequel la silice poreuse se présente sous forme de poudre, de dispersion ou de solide.

3. Procédé selon la revendication 1 ou 2, dans lequel la silice poreuse a un diamètre de particule moyen de 10 à 100 $\mu$m, un diamètre de micropore de 10 à 200 nm, et une surface spécifique de 10 à 200 m$^2$/g.

4. Procédé pour mesurer la lipoprotéine de basse densité dans un échantillon de sang, comprenant :

l'extraction de la lipoprotéine de haute densité d'un échantillon de sang par le procédé selon l'une quelconque des revendications 1 à 3, et
la mesure de la protéine de basse densité dans l'échantillon de sang dont la lipoprotéine de haute densité a été extraite en utilisant (a) de la cholestérol estérase, et (b) de la cholestérol oxydase ou de la cholestérol déshydrogénase.

5. Procédé selon la revendication 4, dans lequel le cholestérol de la lipoprotéine de basse densité est mesuré en laissant la peroxydase et un chromogène agir sur du peroxyde d'hydrogène généré à partir du cholestérol de la lipoprotéine de basse densité par la cholestérol estérase et la cholestérol oxydase, de manière à réaliser une réaction d'apparition de couleur.

6. Procédé selon la revendication 4 ou 5, dans lequel la lipoprotéine de basse densité dans l'échantillon de sang est mesurée en utilisant un élément analytique sec pour mesurer le cholestérol de la lipoprotéine de basse densité, qui comprend (a) de la cholestérol estérase, (b) de la cholestérol oxydase, (c) de la peroxydase, et (d) un chromogène.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 7055812 A
- JP H0755812 A
- JP 6213901 A
- JP H06213901 A
- EP 0627627 A1
- EP 0602455 A2
- US 5409840 A
- EP 0259076 A2
- US 5141872 A
- JP H08113595 A
- JP 60222770 A
- JP S60222770 A
- JP 63219397 A
- JP S63219397 A
- JP 62182652 A
- JP S62182652 A
- JP 55164356 A
- JP S55164356 A
- JP 57066359 A
- JP S5766359 A
- JP 60222769 A
- JP S60222769 A
- JP 57283331 B
- JP S5728333 B
- JP 56142454 U
- JP S56142454 U
- JP 57063452 A
- JP S5763452 A
- JP 58032350 U
- JP S5832350 U
- JP 58501144 A
- JP S58501144 A
- JP 60125543 A
- JP S60125543 A
- JP 60220862 A
- JP S60220862 A
- JP 61294367 A
- JP S61294367 A
- JP 58161867 A
- JP S58161867 A

### Non-patent literature cited in the description

- *Biochemistry,* 1966, vol. 5, 467-477
- **TAKEICHI HORIO et al.** Basic Experimental Methods for Proteins and Enzymes. Nankodo Co., Ltd, 1981